(19) Europäisches Patentamt / European Patent Office / Office européen des brevets

(11) **EP 4 799 572 A1**

(12) **EUROPEAN PATENT APPLICATION**

(43) Date of publication:
**02.09.2026 Bulletin 2026/36**

(21) Application number: **25225559.1**

(22) Date of filing: **19.12.2025**

(51) International Patent Classification (IPC):
***A61B 5/369*** $^{(2021.01)}$ ***A61B 5/00*** $^{(2006.01)}$

(52) Cooperative Patent Classification (CPC):
**A61B 5/7267; A61B 5/369;** A61B 5/327; A61B 5/7203

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **27.02.2025 IN 202521017346**

(71) Applicant: **Tata Consultancy Services Limited**
**Maharashtra (IN)**

(72) Inventors:
- **DATTA, Shreyasi**
  **560066 Bengaluru, Karnataka (IN)**
- **PAL, Arpan**
  **700160 Kolkata, West Bengal (IN)**
- **GUBBI LAKSHMINARASIMHA, Jayavardhana Rama**
  **560066 Bengaluru, Karnataka (IN)**

(74) Representative: **Goddar, Heinz J.**
**Boehmert & Boehmert**
**Anwaltspartnerschaft mbB**
**Pettenkoferstrasse 22**
**80336 München (DE)**

(54) **METHOD AND SYSTEM FOR RECONSTRUCTING NEURO-PHYSIOLOGICAL SIGNALS FROM SINGLE CHANNEL MIXED SIGNALS USING SEPARABLE REPRESENTATIONS**

(57) The growing use of wearable devices requires accurate and compact representations of high dimensional physiological signals. The present disclosure presents a UNet inspired autoencoder to represent and reconstruct multiple neurophysiological signals from single channel data. The architecture includes single-encoder and multiple decoders to obtain self-attention enabled compact embeddings of mixed ExG (EEG /ECG) signals through decaying encoder-decoder skip connections, for improved representation capability. The embeddings are separable into individual ExG components enabling simultaneous reconstruction of high-fidelity EEG and ECG sources. The pretrained encoder can be used for a complex downstream task with minimum fine-tuning. Using the present disclosure on a large corpus of single-channel mixed ExG generated from overnight Polysomnography (PSG) recordings, it is shown that subject- and class- independent EEG/ECG reconstructions validated by multiple domain-specific metrics can be obtained, and the encoded EEG embeddings can be classified into five sleep stages as a downstream task.

Decaying Skip
Mixed @100Hz
3000×1 (T×Ch)
Multi-Head
Self-Attention
Feed
Forward
A
Encoder Blocks
Decoder Blocks
×2
×3
×2
Conv1D
BN
GELU
AvgPool
Conv1D
BN
GELU
Ch+Ch'
Ch'
Ch
T
T'
Stack
Upsample
Conv1D
BN
Leaky
ReLU
Conv
1D
TanH

FIG. 1B



Processed by Luminess, 75001 PARIS (FR)

## Description

CROSS-REFERENCE TO RELATED APPLICATIONS AND PRIORITY

The present application claims priority from Indian application no. 202521017346, filed on February 27, 2025.

TECHNICAL FIELD

The disclosure herein generally relates to the field of biomedical signal processing and, more particularly, to a method and system for reconstructing neuro-physiological signals from single channel mixed signals.

BACKGROUND

Medical wearable industry has grown wider in recent times and these wearable devices are utilized for automated monitoring of neuro-physiological indicators such as stress, anxiety, sleep, etc. of a subject using physiological signals such as Electroencephalogram (EEG)/Electrocardiogram (ECG). Data acquired using wearable device may comprise artifacts and other physiological sources, such as, cardiac, myogenic and ocular signals, especially in highly localized and limited channel settings such as ear-form factors.

Recently, wearable EEG is being explored for continuous home-based settings such as overnight sleep quality estimation. The clinical gold standard for assessing sleep quality is Polysomnography (PSG), where EEG, ECG, EMG, and EOG are segmented into wake (W), Rapid Eye Movement (R) and non-REM (N1, N2, N3) sleep stages using clinical guidelines. Automated sleep staging methods primarily use EEG features for supervised classification of epoched overnight data. Initial deep learning architectures in this area employed CNNs and RNNs to capture short input contexts, thereby being limited in modelling temporal dependencies essential for sleep-stage transitions. This led to the emergence of sequence-to-sequence hybrid models comprising epoch encoders to transform input epochs into features (e.g., CNNs, ResNet, UNet, RNNs, transformers) and sequence encoders to transform these features across epochs into sleep stage sequences (e.g., RNNs, self-attention and transformers).

Conventional techniques for reconstructing the physiological signals requires multiple mixed signal channels from independent sources and are not generic enough to tune and scale to large datasets and diverse applications. Deep learning-based techniques are being introduced for high fidelity physiological signal reconstructions along with effective representations, as a necessary pre-processing step for resource limited computing. End-to-end representation learning models achieve this high-fidelity physiological signal by projecting input signals to a compact embedding space, while reconstructing or generating new data from the learnt abstraction. Most of these existing methods use generative modelling for reconstructing physiological signals, but these methods are often fixated on irrecoverable information rather than recovering/separating input physiological signals. Existing works on deterministic deep learning models for EEG/ECG reconstructions are usually limited to EEG denoising only, and do not reconstruct other physiological sources, limiting significant domain information recovery.

SUMMARY

Embodiments of the present disclosure present technological improvements as solutions to one or more of the above-mentioned technical problems recognized by the inventors in conventional systems. For example, in one embodiment, a method for reconstructing neuro-physiological signals from single channel mixed signals is provided. The method includes receiving, by one or more hardware processors, a signal from a sensor based wearable device worn by a subject under test for monitoring health conditions, wherein the received signal is a mixed signal comprising a plurality of neuro-physiological signals. Further, the method includes generating, by the one or more hardware processors an encoded joint embedding of the received signal using a single encoder associated with a trained autoencoder network, wherein the single encoder comprises a plurality of convolution blocks, wherein each of the plurality of convolution blocks comprises a plurality of convolutions followed by batch normalizations, activation layers, and a pooling layer. Furthermore, the method includes generating, by the one or more hardware processors, a final separable embedding based on an aggregated temporal context associated with the received signal by passing the encoded joint embeddings through additional convolutions, a Multi-Head Attention (MHA) mechanism and a feed forward layer associated with the trained autoencoder network, wherein the feed forward layer uses residual connections and layer normalizations. Finally, the method includes generating, by the one or more hardware processors, a plurality of reconstructed signals comprising at least one of a plurality of neural signals and a plurality of physiological signals based on the final separable embedding of the received signal using a multiple decoder framework associated with the trained autoencoder network, by: (i) generating a plurality of decoded signals by performing linear up sampling followed by a plurality of convolutions, batch normalizations and activations based on the final separable embedding using a plurality of decoders associated with the

multiple decoder framework (ii) generating a plurality of potential reconstructed signals based on the plurality of decoded signals using additional convolution layers associated with the multiple decoder framework (iii) predicting a residual error associated with each of the plurality of the potential reconstructed signals using final convolution layers associated with the multiple decoder framework and (iv) generating the plurality of reconstructed signals by adding an associated predicted residual error to each of the plurality of potential reconstructed signals.

In another aspect, a system for reconstructing neuro-physiological signals from single channel mixed signals is provided. The system includes at least one memory storing programmed instructions, one or more Input /Output (I/O) interfaces, and one or more hardware processors operatively coupled to the at least one memory, wherein the one or more hardware processors are configured by the programmed instructions to receive a signal from a sensor based wearable device worn by a subject under test for monitoring health conditions, wherein the received signal is a mixed signal comprising a plurality of neuro-physiological signals. Further, the one or more hardware processors are configured by the programmed instructions to generate an encoded joint embedding of the received signal using a single encoder associated with a trained autoencoder network, wherein the single encoder comprises a plurality of convolution blocks, wherein each of the plurality of convolution blocks comprises a plurality of convolutions followed by batch normalizations, activation layers, and a pooling layer. Furthermore, the one or more hardware processors are configured by the programmed instructions to generate a final separable embedding based on an aggregated temporal context associated with the received signal by passing the encoded joint embeddings through additional convolutions, a Multi-Head Attention (MHA) mechanism and a feed forward layer associated with the trained autoencoder network, wherein the feed forward layer uses residual connections and layer normalizations. Finally, the one or more hardware processors are configured by the programmed instructions to generate a plurality of reconstructed signals comprising at least one of a plurality of neural signals and a plurality of physiological signals based on the final separable embedding of the received signal using a multiple decoder framework associated with the trained autoencoder network, by: (i) generating a plurality of decoded signals by performing linear up sampling followed by a plurality of convolutions, batch normalizations and activations based on the final separable embedding using a plurality of decoders associated with the multiple decoder framework (ii) generating a plurality of potential reconstructed signals based on the plurality of decoded signals using additional convolution layers associated with the multiple decoder framework (iii) predicting a residual error associated with each of the plurality of the potential reconstructed signals using final convolution layers associated with the multiple decoder framework and (iv) generating the plurality of reconstructed signals by adding an associated predicted residual error to each of the plurality of potential reconstructed signals.

In yet another aspect, there are provided one or more non-transitory machine-readable information storage mediums comprising one or more instructions which when executed by one or more hardware processors cause: receive a signal from a sensor based wearable device worn by a subject under test for monitoring health conditions, wherein the received signal is a mixed signal comprising a plurality of neuro-physiological signals. Further, the one or more non-transitory machine-readable information storage mediums comprising one or more instructions which when executed by one or more hardware processors cause generating an encoded joint embedding of the received signal using a single encoder associated with a trained autoencoder network, wherein the single encoder comprises a plurality of convolution blocks, wherein each of the plurality of convolution blocks comprises a plurality of convolutions followed by batch normalizations, activation layers, and a pooling layer. Furthermore, the one or more non-transitory machine-readable information storage mediums comprising one or more instructions which when executed by one or more hardware processors cause generating a final separable embedding based on an aggregated temporal context associated with the received signal by passing the encoded joint embeddings through additional convolutions, a Multi-Head Attention (MHA) mechanism and a feed forward layer associated with the trained autoencoder network, wherein the feed forward layer uses residual connections and layer normalizations. Finally, the one or more non-transitory machine-readable information storage mediums comprising one or more instructions which when executed by one or more hardware processors cause generating a plurality of reconstructed signals comprising at least one of a plurality of neural signals and a plurality of physiological signals based on the final separable embedding of the received signal using a multiple decoder framework associated with the trained autoencoder network, by: (i) generating a plurality of decoded signals by performing linear up sampling followed by a plurality of convolutions, batch normalizations and activations based on the final separable embedding using a plurality of decoders associated with the multiple decoder framework (ii) generating a plurality of potential reconstructed signals based on the plurality of decoded signals using additional convolution layers associated with the multiple decoder framework (iii) predicting a residual error associated with each of the plurality of the potential reconstructed signals using final convolution layers associated with the multiple decoder framework and (iv) generating the plurality of reconstructed signals by adding an associated predicted residual error to each of the plurality of potential reconstructed signals.

It is to be understood that both the foregoing general description and the following detailed description are exemplary and explanatory only and are not restrictive of the invention, as claimed.

## BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1A is a functional block diagram of a system for reconstructing neuro-physiological signals from single channel mixed signals, in accordance with some embodiments of the present disclosure.
FIG. 1B and FIG. 1C illustrates overall functional architecture of the system for the reconstructing neuro-physiological signals from single channel mixed signals, in accordance with some embodiments of the present disclosure.
FIG. 2 illustrates a flow diagram for a processor implemented method for reconstructing neuro-physiological signals from single channel mixed signals, in accordance with some embodiments of the present disclosure.
FIG. 3A through FIG. 3F illustrates experimental results for the processor implemented method for reconstructing neuro-physiological signals from single channel mixed signals, in accordance with some embodiments of the present disclosure.

## DETAILED DESCRIPTION OF EMBODIMENTS

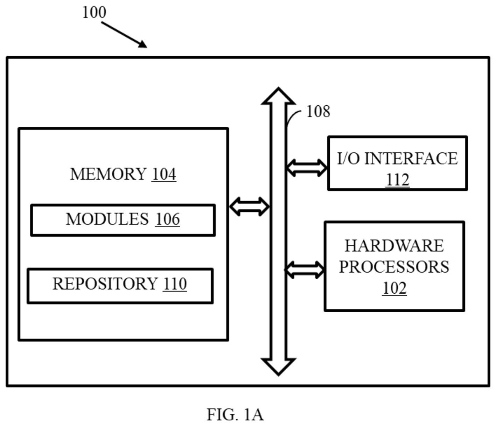

Exemplary embodiments are described with reference to the accompanying drawings. In the figures, the left-most digit(s) of a reference number identify the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.
Most of these existing methods use generative modelling for reconstructing physiological signals, however these methods are often fixated on irrecoverable information rather than recovering/separating input physiological signals. Existing works on deterministic deep learning models for physiological signal reconstructions are usually limited to EEG denoising only, and do not reconstruct other physiological sources, limiting significant domain information recovery.
To overcome the challenges of the conventional approaches, embodiments herein provide a method and system for reconstructing neuro-physiological signals from single channel mixed signals.
The present disclosure presents an autoencoder framework for multiple signal source reconstruction and representation, with a plurality of complex downstream applications like sleep staging. The contributions of the present disclosure includes (i) a single encoder-multiple decoder, UNet inspired autoencoder architecture, to obtain self-attention enabled compact and separable embeddings of fixed-length single-channel mixed signal segments for simultaneous reconstruction of neuro-physiological signals like Electroencephalogram (EEG), Electrocardiogram (ECG) and the like (ii) introducing gradual decay of encoder-decoder skip connections to enable a balance between high fidelity reconstruction and accurate representations and (iii) complex downstream applications like sleep stage segmentation using generated separable embeddings with minimum retraining of the encoder.
Referring now to the drawings, more particularly to FIG. 1A through FIG. 3F, where similar reference characters denote corresponding features consistently throughout the figures, there are shown preferred embodiments, and these embodiments are described in the context of the following exemplary system and/or method.
FIG. 1A is a functional block diagram of system 100 for reconstructing neuro-physiological signals from single channel mixed signals, in accordance with some embodiments of the present disclosure. The system 100 includes or is otherwise in communication with hardware processors 102, at least one memory such as a memory 104, an Input /Output (I/O) interface 112. The hardware processors 102, memory 104, and the I/O interface 112 may be coupled by a system bus such as a system bus 108 or a similar mechanism. In an embodiment, the hardware processors 102 can be one or more hardware processors.
The I/O interface 112 may include a variety of software and hardware interfaces, for example, a web interface, a graphical user interface, and the like. The I/O interface 112 may include a variety of software and hardware interfaces, for example, interfaces for peripheral device(s), such as a keyboard, a mouse, an external memory, a printer and the like. Further, the I/O interface 112 may enable system 100 to communicate with other devices, such as web servers, and external databases.
The I/O interface 112 can facilitate multiple communications within a wide variety of networks and protocol types, including wired networks, for example, local area network (LAN), cable, etc., and wireless networks, such as Wireless LAN (WLAN), cellular, or satellite. For the purpose, the I/O interface 112 may include one or more ports for connecting several computing systems with one another or to another server computer. The I/O interface 112 may include one or more ports for connecting several devices to one another or to another server.
The one or more hardware processors 102 may be implemented as one or more microprocessors, micro-computers, microcontrollers, digital signal processors, central processing units, node machines, logic circuitries, and/or any devices that manipulate signals based on operational instructions. Among other capabilities, the one or more

hardware processors 102 is configured to fetch and execute computer-readable instructions stored in memory 104.

The memory 104 may include any computer-readable medium known in the art including, for example, volatile memory, such as static random-access memory (SRAM) and Dynamic Random Access Memory (DRAM), and/or nonvolatile memory, such as read only memory (ROM), erasable programmable ROM, flash memories, hard disks, optical disks, and magnetic tapes. In an embodiment, memory 104 includes a plurality of modules 106. Memory 104 also includes a data repository (or repository) 110 for storing data processed, received, and generated by the plurality of modules 106.

The plurality of modules 106 includes programs or coded instructions that supplement applications or functions performed by the system 100 for reconstructing neuro-physiological signals from single channel mixed signals. The plurality of modules 106, amongst other things, can include routines, programs, objects, components, and data structures, which perform particular tasks or implement particular abstract data types. The plurality of modules 106 may also be used as, signal processor(s), node machine(s), logic circuitries, and/or any other device or component that manipulates signals based on operational instructions. Further, the plurality of modules 106 can be used by hardware, by computer-readable instructions executed by the one or more hardware processors 102, or by a combination thereof. The plurality of modules 106 can include various sub-modules (not shown). The plurality of modules 106 may include computer-readable instructions that supplement applications or functions performed by the system 100 for Reconstructing neuro-physiological signals from single channel mixed signals.

The data repository (or repository) 110 may include a plurality of abstracted pieces of code for refinement and data that is processed, received, or generated as a result of the execution of the plurality of modules in the module(s) 106.

Although the data repository 110 is shown internal to the system 100, it will be noted that, in alternate embodiments, the data repository 110 can also be implemented external to the system 100, where the data repository 110 may be stored within a database (repository 110) communicatively coupled to the system 100. The data contained within such an external database may be periodically updated. For example, new data may be added into the database (not shown in FIG. 1A) and/or existing data may be modified and/or non-useful data may be deleted from the database. In one example, the data may be stored in an external system, such as a Lightweight Directory Access Protocol (LDAP) directory, a Relational Database Management System (RDBMS).

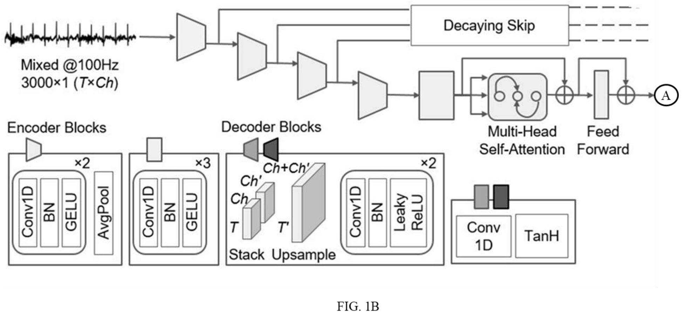

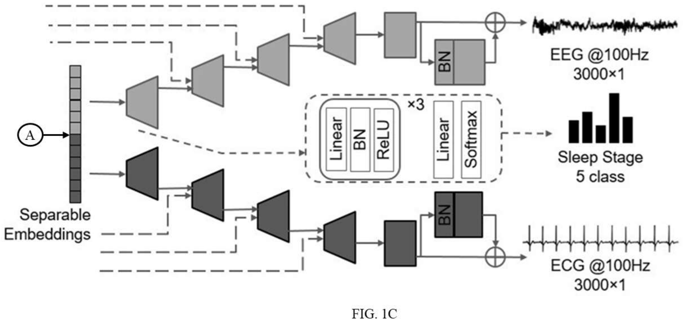

The overall architecture of the system of FIG. 1A is explained in conjunction with FIG. 1B and FIG. 1C. For example, the architecture shown in FIG.1B and FIG. 1C is a UNet inspired autoencoder architecture with a single encoder framework (or single encoder) and multiple decoder framework with a plurality of branched decoders to process 30-sec epochs of mixed neuro-physiological signals like EEG and ECG. The encoder projects the mixed input to a joint embedding space, passing it through Multi-Head Attention (MHA) to encode temporal context. The embedding dimensions are separated into respective modalities like EEG and ECG modalities, such that, each decoder learns the mapping of their corresponding representations back to the individual signal space. Due to the embedding separability, only selected (EEG) embeddings can now be passed to a fully connected network (FCN) for a downstream sleep stage classification task, requiring minimal encoder retraining.

The single encoder includes four convolution blocks, each with 2 sets of 1D convolution with kernels of size 5, followed by batch normalization (BN), activation (GELU) and average pooling in order. Pooling windows of 2, 2, 5, and 5, respectively in these four blocks reduces the input dimensionality by a factor of 100. Three additional convolutions with kernel size 3 are applied after all down sampling to further refine the embeddings of the mixed signal.

Further, the encoded joint embeddings are passed through a MHA layer comprising a self-attention block with 4 heads to aggregate temporal context. The final embedding is produced by a feed forward (FF) layer. This attention and FF layout makes use of residual connections and layer normalizations as in add-and-norm layers of the transformer encoder architecture, for propagating stronger gradients to earlier layers. Each of the plurality of neuro-physiological signal embeddings are then sent separately to their respective decoders. Keeping parity with the encoder blocks, each decoder block performs linear up sampling followed by two sets of convolution, batch normalization and activation (Leaky-ReLU) in order. A final convolution reconstructs the signal, while an additional convolution layer takes this reconstruction and predicts the residual errors. The errors are added back to the preliminary reconstruction to arrive at the final reconstructed signal.

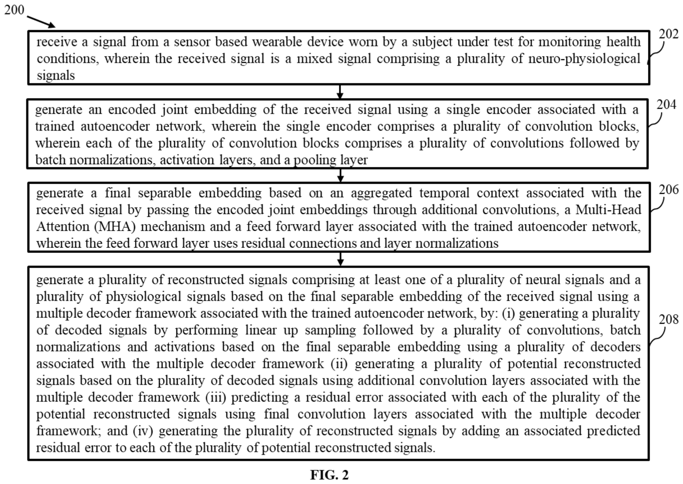

The working of the components of system 100 are explained with reference to the method steps depicted in FIG. 2.

FIGS. 2 is an exemplary flow diagram illustrating a method 200 for reconstructing neuro-physiological signals from single channel mixed signals implemented by the system of FIG. 1A and 1B, according to some embodiments of the present disclosure. In an embodiment, the system 100 includes one or more data storage devices or the memory 104 operatively coupled to the one or more hardware processor(s) 102 and is configured to store instructions for execution of steps of the method 200 by the one or more hardware processors 102. The steps of method 200 of the present disclosure will now be explained with reference to the components or blocks of system 100 as depicted in FIG. 1A and 1B and the steps of flow diagram as depicted in FIG. 2. The method 200 may be described in the general context of computer executable instructions. Generally, computer executable instructions can include routines, programs, objects, compo-

nents, data structures, procedures, modules, functions, etc., that perform particular functions or implement particular abstract data types.

The method 200 may also be practiced in a distributed computing environment where functions are performed by remote processing devices that are linked through a communication network. The order in which steps of the method 200 is described is not intended to be construed as a limitation, and any number of the described method blocks can be combined in any order to implement the method 200, or an alternative method. Furthermore, the method 200 can be implemented in any suitable hardware, software, firmware, or combination thereof.

Now referring to FIG. 2, at step 202 of method 200, the one or more hardware processors 102 are configured by the programmed instructions to receive a signal from a sensor based wearable device worn by a subject under test for monitoring health conditions, wherein the received signal is a mixed signal comprising a plurality of neuro-physiological signals.

At step 204 of the method 200, the one or more hardware processors 102 are configured by the programmed instructions to generate an encoded joint embedding of the received signal using a single encoder associated with a trained autoencoder network, wherein the single encoder comprises a plurality of convolution blocks, wherein each of the plurality of convolution blocks comprises a plurality of convolutions followed by batch normalizations, activation layers, and a pooling layer.

At step 206 of the method 200, the one or more hardware processors 102 are configured by the programmed instructions to generate a final separable embedding based on an aggregated temporal context associated with the received signal by passing the encoded joint embeddings through additional convolutions, a Multi-Head Attention (MHA) mechanism and a feed forward layer associated with the trained autoencoder network, wherein the feed forward layer uses residual connections and layer normalizations. The final separable embedding includes a plurality of embeddings pertaining to each of the plurality of neuro-physiological signals.

Now, referring back to FIG. 2, at step 208 of the method 200, the one or more hardware processors 102 are configured by the programmed instructions to generate a plurality of reconstructed signals comprising at least one of a plurality of neural signals and a plurality of physiological signals based on the final separable embedding of the received signal using a multiple decoder framework associated with the trained autoencoder network. The plurality of reconstructed signals includes a plurality of neural signals and/or a plurality of physiological signals

For example, steps for generating the plurality of reconstructed signals is explained below: Initially, a plurality of decoded signals are generated by performing linear up sampling followed by a plurality of convolutions, batch normalizations and activations based on the final separable embedding using a plurality of decoders associated with the multiple decoder framework of FIG. 1B. Further, a plurality of potential reconstructed signals are generated based on the plurality of decoded signals using additional convolution layers associated with the multiple decoder framework. Post generating the plurality of potential reconstructed signals, a residual error associated with each of the plurality of the potential reconstructed signals are predicted using final convolution layers associated with the multiple decoder framework. Finally, the plurality of reconstructed signals are generated by adding an associated predicted residual error to each of the plurality of potential reconstructed signals.

**Training:** For example, the autoencoder network comprises the single encoder with the MHA and the feed forward layer and a multiple decoder framework comprising the plurality of decoders is trained as follows: Initially, the autoencoder network with a plurality of skip connections between the single encoder and the plurality of decoders is trained until a predefined number of training epochs to learn an encode function $f_{enc}$ to encode a plurality of input signals $x_{ExG}$ into an associated plurality of separable embeddings z = [$z_{eeg}$, $z_{ecg}$, ...... ]. The plurality of input signals are mixed signals including the plurality of neuro-physiological signals.

For example, the autoencoder model starts training with skip connections, but the skips decay as training progresses. This is achieved through the use of dropout, with linearly increasing dropout rates leading to minimal skip connections within the first few epochs. Fuller skip connections in the early phase of training allows the initial solution to get closer to the final optimum, while the absence of skips later ensures that high frequency information is captured in the encoded embedding eventually. The learnt physiological signal embeddings are flattened and passed through the FCN comprising consecutive linear, batch normalization and activation (ReLU) layers, followed by a linear and softmax activation layer, to identify sleep stages as a separate downstream task. This classification network has the provision for retraining a selected number of layers of the trained encoder to fit to a downstream task.

Further, the autoencoder network is trained until a predefined number of training epochs to learn a plurality of decode functions to map the associated plurality of separable embeddings to a plurality of reconstructed input signals, wherein the plurality of skip connections between single encoder and the plurality of decoders decays for a predefined number of epochs as the training progresses.

The decaying of skip connections is performed by increasing dropout leading to minimal skip connections within the first few epochs. Further, the decaying of skip connections allows the autoencoder to learn high frequency information for the reconstruction of the plurality of input signals and also producing accurate representations of the input signals in the respective encoded embeddings. The respective encoded embeddings or the plurality of separable embeddings

associated with each of the plurality of reconstructed signals are utilized to perform the plurality of downstream task with minimal finetuning of the encoder in the trained autoencoder network and, wherein the plurality of downstream task comprises automated sleep-stage classification.

Further, the autoencoder network is trained to generate a plurality of reconstructed signals associated with the plurality of input signals based on the associated plurality of separable embeddings using a multimodal reconstruction loss. The multimodal reconstruction loss is a weighted sum of smooth L1-loss and a correlation-based loss between each of the plurality of reconstructed signals from each of the plurality of decoder and an associated input signal from among the plurality of input signals. Finally, the plurality of reconstructed signals are validated based on an associated plurality of parameters.

The goal for reconstruction is to learn the function $f_{enc}$ to encode the mixed input $x_{ExG}$ into the joint embedding space z = [$z_{eeg}$, $z_{ecg}$,......]., and $f_{dec}^{1}, f_{dec}^{2} \;\ldots\ldots f_{dec}^{n}$ to map z back to the individual reconstructed components $\tilde{x}_{EEG}$, $\tilde{x}_{ECG}$ ..........as shown in equations (1) and (2), $\theta_{enc}$, $\theta_{dec}^{1}$ and $\theta_{dec}^{2}$ being the learnable network parameters, using an ensemble of loss functions.

$$z = f_{enc}(x_{ExG},\ \theta_{enc}) \ldots\ldots\ldots\ldots\ldots\ldots\ldots\ldots (1)$$

$$\tilde{x}_{EEG} = f_{dec}^{1}(z_{eeg}, \theta_{dec}^{1}),\ \tilde{x}_{ECG} = f_{dec}^{2}(z_{ecg}, \theta_{dec}^{2}) \ldots\ldots\ldots\ldots\ldots\ldots\ldots (2)$$

The multimodal reconstruction loss $L_{rec}$ shown in (3) is a weighted sum (weights $\alpha_i$, $i \in [1, 4]$) of smooth L1-loss $L_{sL1}$ and correlation based loss $L_{PCC}$ between the original and predicted unimodal signals from each of the plurality of decoders. The former is a combination of MSE and L1 losses with reduced sensitivity to incidental temporal outliers in the *ExG*. The latter, computed as the squared deviation of Pearson Correlation Coefficient $\rho$ from maximum correlation of unity between original and reconstructed signals as shown in (4), is important for reconstructing high frequency components.

$$L_{rec} = \alpha_1 L_{sL1}(x_{EEG}, \tilde{x}_{EEG}) + \alpha_2 L_{sL1}(x_{ECG}, \tilde{x}_{ECG}) + \alpha_3 L_{PCC}(x_{EEG}, \tilde{x}_{EEG}) + \alpha_4 L_{PCC}(x_{ECG}, \tilde{x}_{ECG}) \quad (3)$$

$$L_{PCC}(x, \tilde{x}) = (1 - \rho)^2 \ldots\ldots\ldots\ldots\ldots\ldots\ldots\ldots\ldots\ldots\ldots\ldots\ldots\ldots (4)$$

Reconstruction of the plurality of neuro-physiological signals is evaluated based on the associated parameters. For example, the EEG signals are evaluated by relative power differences in four frequency bands $\Delta\delta$ (0.5 - *4 Hz*), $\Delta\theta$ (4 - 8 *Hz*), $\Delta\alpha$ (8 - *13 Hz*), $\Delta\beta$ (13 - 30 *Hz*) and all bands $\Delta P$, signal-to-noise ratio *SNR* (ratio of RMS of true to RMS error between true and predicted signals, dB). Absolute differences in Heart Rate (HR) $\Delta hr$ (beats/min), HR variability $\Delta$hrv (s) for ECG, and time-domain correlation coefficient $\rho$ for both signals are used for validation. For the downstream classification task, the present disclosure utilizes a weighted class-aware multi-class cross entropy loss to account for unbalanced class distributions in sleep stages.

Data Preparation and Implementation: Mixed ExG signals are generated by linearly mixing pure EEG and ECG segments using (equation 5), where $\lambda_1$ and $\lambda_2$ are scaling hyperparameters ($0 < \lambda_1, \lambda_2 < 1$) to control SNR (equation 6) in the mixed ExG signal.

$$x_{ExG} = \lambda_1 x_{EEG} + \lambda_2 x_{ECG} \ldots\ldots\ldots\ldots\ldots\ldots (5)$$

$$SNR = 10 \log \frac{(\lambda_1 x_{EEG})}{(\lambda_2 x_{ECG})} \ldots\ldots\ldots\ldots\ldots\ldots\ldots\ldots (6)$$

For example, the present disclosure utilized ECG and C3 lead EEG from overnight PSG recordings of 994 subjects of the publicly available Physionet Challenge 2018 dataset. Here, 623 subjects are used for training (*train*), 198 for validation (*eval*), and kept 173 separate for testing (*test*). Each individual raw EEG/ECG signal was down sampled to 100 Hz, filtered using a 45 Hz low-pass filter, and epoched into 30-sec windows with one corresponding sleep stage label belonging to {W, R, N1, N2, N3}. All epochs undefined beyond these 5 stages were excluded. Any outlier beyond median ± 5 * sd of the global EEG amplitude was flagged, and an epoch containing more than 1% of these flags were excluded. For each epoch, individual signals were standardized between [-1, 1] range, scaled by $\lambda 1$ or $\lambda 2$ drawn randomly from uniform distributions between [0.4, 0.5] and [0.5, 0.6] respectively (ensuring higher ECG artifacts), and detrended by mean

subtraction prior to mixing.

Experiments were run for batch size of 128 for 100 epochs for both reconstruction and downstream tasks. For reconstruction, the weight of each encoder-decoder skip connection is decayed from 100% to *X*% until 25th epoch. The present disclosure is experimented with full 100% skip throughout without any dropout (FS), skips decayed to *X*=20%, 10% (20S, 10S) and *X*=0% or no-skip (NS) configurations. For the downstream task, last 20 (out of 44) pretrained encoder layers were re-trained before passing the EEG embeddings to the FCN. Convolution weights in the encoder and decoder, and linear layer weights in the FCN were initialized from normal and Kaiming normal distributions respectively. The loss coefficients in (3) were determined heuristically. For both tasks, Adam optimizer ($\beta_1 = 0.9$, $\beta_2 = 0.999$, $\varepsilon = 10^{-8}$) was used with an adaptive learning rate. All experiments were implemented in Python 3.8.10 with PyTorch 2.3.1 library, running on an NVIDIA RTX A6000 GPU.

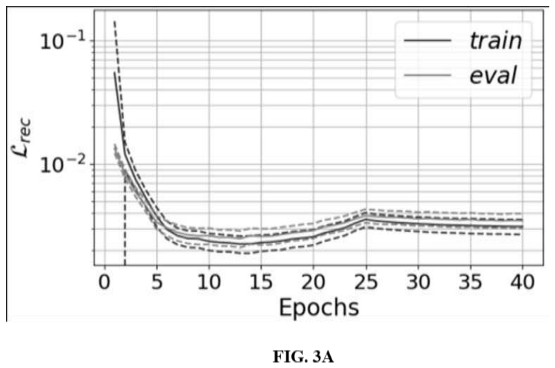

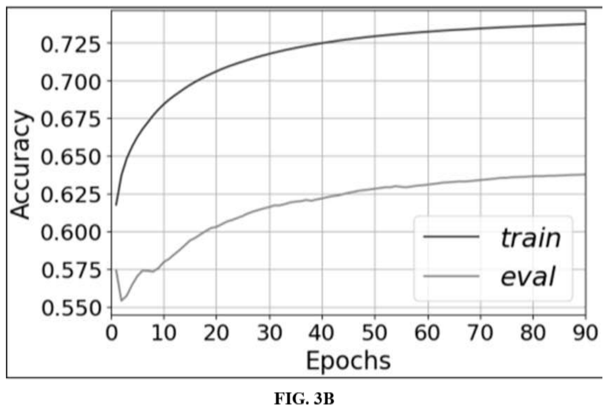

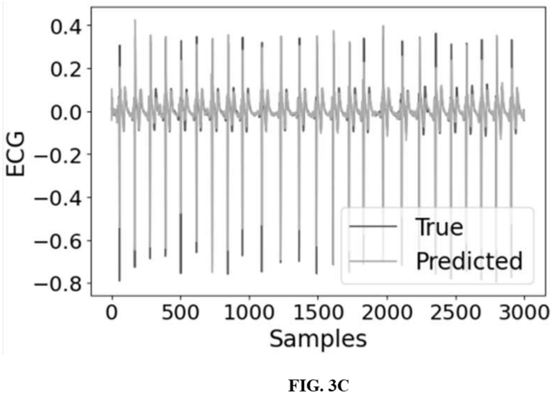

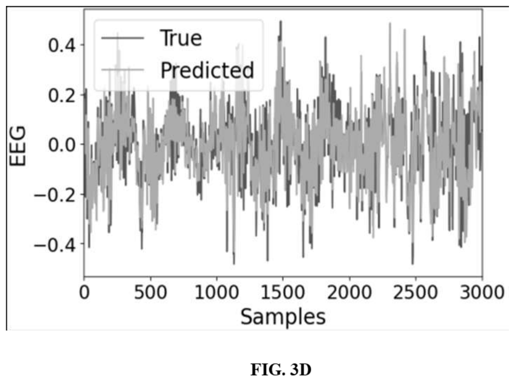

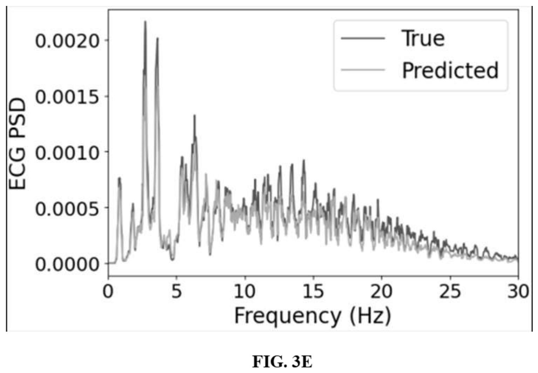

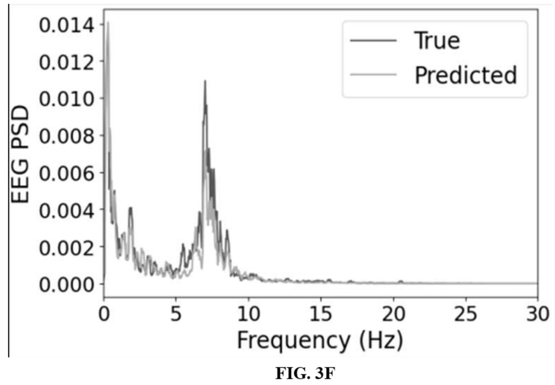

**Results and discussions:** The epoch-wise variation of reconstruction loss and classification accuracy during training for 20S are illustrated in FIG. 3A and 3B respectively showing convergence in both tasks. $L_{rec}$ slightly increases after the initial rapid decrease due to linearly decaying skips upto epoch 25. The experiments demonstrate that decaying skips leads to quicker initial reconstruction convergence than using no skip connections, while producing better embeddings than using full skips. Optimum balance between reconstruction and representation was found for 20S. Examples of reconstructed EEG and ECG (shown in FIGS 3C to 3E) for this configuration from test show significant similarity in the input and predicted signals in both time domain and Power Spectral Density (PSD) of frequency domain.

Quantitative metrics for reconstruction on test are shown in Table 1. For EEG, FS shows highest reconstruction quality with small power differences in all bands, high mean SNR compared to input mixed data (SNR for input mixed test = - 0.60 ± 3.33 dB), and high $\rho$ > 90% between true and predicted signals. The metrics deteriorate for further reduction in skip weights in 10S and NS, with $\rho$ falling below 85%. Band powers in higher frequencies are more affected as seen from $\Delta P$ and $\Delta\beta$. However, 20S retains a decent performance with $\rho$ > 85% though following similar trend of higher errors in the higher frequency bands. ECG reconstructions show high accuracy throughout, with < 2 beats/min $\Delta$hr and $\rho$ > 80% in all configurations. It is comparatively lesser affected as skip connections are completely removed. As a comparison, the vanilla denoising UNet (IC-UNet) with losses implemented as on our train and eval set produced slightly improved test metrics ($\Delta\delta$, $\Delta\theta$, $\Delta\alpha$, $\Delta\beta$, $\Delta P$, of 0.08 ± 0.20, 0.03 ± 0.05, 0.01 ± 0.03, 0.04 ± 0.29, 0.13 ± 0.39, and $\rho$ = 0.89 ± 0.09) at the cost of higher embedding dimensions and only EEG reconstructions.

**Table 1**

| Metrics | | FS | 20S | 10S | NS |
|---|---|---|---|---|---|
| EEG | $\Delta\delta$ | 0.04 ± 0.05 | 0.08 ± 0.13 | 0.07 ± 0.13 | 0.09 ± 0.14 |
| | $\Delta\theta$ | 0.02 ± 0.04 | 0.05 ± 0.05 | 0.06 ± 0.05 | 0.12 ± 0.07 |
| | $\Delta\alpha$ | 0.02 ± 0.03 | 0.04 ± 0.05 | 0.06 ± 0.06 | 0.11 ± 0.09 |
| | $\Delta\beta$ | 0.04 ± 0.12 | 0.07 ± 0.09 | 0.09 ± 0.10 | 0.09 ± 0.10 |
| | $\Delta P$ | 0.08 + 0.26 | 0.26 ± 0.21 | 0.27 ± 0.21 | 0.42 ± 0.25 |
| | $\rho$ | 0.94 ± 0.07 | 0.86 ± 0.09 | 0.81 ± 0.12 | 0.76 ± 0.15 |
| | SNR | 5.72 ± 1.94 | 3.39 ± 1.16 | 2.81 ± 1.17 | 2.28 ± 1.24 |
| ECG | $\Delta hr$ | 0.06 ± 3.92 | 1.16 ± 5.12 | 1.97 + 6.23 | 1.63 ± 7.70 |
| | $\Delta hrv$ | 0.004 ± 0.02 | 0.01 ± 0.03 | 0.02 ± 0.04 | 0.01 ± 0.03 |
| | $\rho$ | 0.96 ± 0.05 | 0.88 ± 0.10 | 0.83 ± 0.12 | 0.83 ± 0.18 |

Classification performance in the downstream task are presented in Table 2, showing 5-class overall Accuracy (Acc) and average F1-score (F1). Following the expected trend, performance improves with decaying skip connections to 20S compared to full skips. No significant difference was observable for reducing the skip weights further. Analysis of the EEG embeddings retrained for the classification task using the pretrained encoder with FCN for initial and almost final training epochs showed that the embeddings are already well separated with minimal fine tuning on the class labels. Adding a few additional fully connected layers, and training further tunes them to the complex classification task with F1 across 5 classes reaching [0.70, 0.45, 0.40, 0.75, 0.71] for {W, R, N1, N2, N3} respectively. Lower performance in N1 and R occurs due to harder separability and smaller number of training samples for these classes. As comparison, EEG embeddings from retrained IC-UNet (as above) produced overall Acc and F1 of 0.47 and 0.35 respectively on unseen test. UTime reported cross-validation F1 of 0.77 for the same dataset, however this was a dedicated coarse one-pass pure EEG segmentation task, without the constraints of high fidelity source reconstruction or easily tunable embedding dimensions.

Table II

| Metrics | FS | 20S | 10S | NS |
|---|---|---|---|---|
| Acc | 0.59 | 0.65 | 0.65 | 0.65 |
| F1 | 0.53 | 0.60 | 0.59 | 0.60 |

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

The embodiments of the present disclosure herein address the unresolved problem of reconstructing neuro-physiological signals from single channel mixed signals. The present disclosure presented an autoencoder with dual-branched decoders and decaying encoder-decoder skips to separate EEG-ECG signals from single channel mixtures, while also enabling rich latent representations. The model, trained on a large corpus and demonstrating generalization to unseen test data, extracts features tunable for sleep-staging by retraining only few encoder layers and a simple fully connected layer.

It is to be understood that the scope of the protection is extended to such a program and in addition to a computer-readable means having a message therein such computer-readable storage means contain program-code means for implementation of one or more steps of the method when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g. an ASIC and an FPGA, or at least one microprocessor and at least one memory with software modules located therein. Thus, the means can include both hardware means, and software means. The method embodiments described herein could be implemented in hardware and software. The device may also include software means. Alternatively, the embodiments may be implemented on different hardware devices, e.g., using a plurality of CPUs, GPUs and edge computing devices.

The embodiments herein can comprise hardware and software elements. The embodiments that are implemented in software include but are not limited to, firmware, resident software, microcode, etc. The functions performed by various modules described herein may be implemented in other modules or combinations of other modules. For the purposes of this description, a computer-usable or computer readable medium can be any apparatus that can comprise, store, communicate, propagate, or transport the program for use by or in connection with the instruction execution system, apparatus, or device. The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items or meant to be limited to only the listed item or items. It must also be noted that as used herein and in the appended claims, the singular forms "a," "an," and "the" include plural references unless the context clearly dictates otherwise. Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e. non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A processor-implemented method (200), the method comprising:

    receiving (202), by one or more hardware processors, a signal from a sensor based wearable device worn by a subject under test for monitoring health conditions, wherein the received signal is a mixed signal comprising a plurality of neuro-physiological signals;
    generating (204), by the one or more hardware processors, an encoded joint embedding of the received signal using a single encoder associated with a trained autoencoder network, wherein the single encoder comprises a plurality of convolution blocks, wherein each of the plurality of convolution blocks comprises a plurality of convolutions followed by batch normalizations, activation layers, and a pooling layer;
    generating (206), by the one or more hardware processors, a final separable embedding based on an aggregated temporal context associated with the received signal by passing the encoded joint embeddings through additional convolutions, a Multi-Head Attention (MHA) mechanism and a feed forward layer associated with the trained autoencoder network, wherein the feed forward layer uses residual connections and layer normalizations; and
    generating (208), by the one or more hardware processors, a plurality of reconstructed signals comprising at least one of a plurality of neural signals and a plurality of physiological signals based on the final separable embedding of the received signal using a multiple decoder framework associated with the trained autoencoder network, by:

        generating a plurality of decoded signals by performing linear up sampling followed by a plurality of convolutions, batch normalizations and activations based on the final separable embedding using a plurality of decoders associated with the multiple decoder framework;
        generating a plurality of potential reconstructed signals based on the plurality of decoded signals using additional convolution layers associated with the multiple decoder framework;
        predicting a residual error associated with each of the plurality of the potential reconstructed signals using final convolution layers associated with the multiple decoder framework; and
        generating the plurality of reconstructed signals by adding an associated predicted residual error to each of the plurality of potential reconstructed signals.

2. The method as claimed in claim 1, wherein the final separable embedding comprises a plurality of separable embeddings pertaining to each of the plurality of neuro-physiological signals.

3. The method as claimed in claims 1, wherein the trained autoencoder network comprises the single encoder with the MHA and the feed forward layer and a multiple decoder framework comprising the plurality of decoders and convolution layers, wherein steps for training the autoencoder network comprises:

    initializing training of the autoencoder network with a plurality of skip connections between the single encoder and the plurality of decoders until a predefined number of training epochs to learn an encode function to encode a plurality of input signals into an associated plurality of separable embeddings, wherein the plurality of input signals are mixed signals comprising the plurality of neuro-physiological signals;
    training the autoencoder network until a predefined number of training epochs to learn a plurality of decode functions to map the associated plurality of separable embeddings to a plurality of input signals, wherein the plurality of skip connections between single encoder and the plurality of decoders decays for a predefined number of epochs as the training progresses, wherein decaying of skip connections is performed by increasing dropout leading to minimal skip connections within the first few epochs and, wherein the decaying of skip connections allows the autoencoder to learn high frequency information for the reconstruction of the plurality of input signals and also producing accurate representations of the input signals in the respective encoded embeddings;
    training autoencoder network to generate a plurality of reconstructed signals associated with the plurality of input signals based on the associated plurality of separable embeddings using a multimodal reconstruction loss, wherein the multimodal reconstruction loss is a weighted sum of smooth L1-loss and a correlation-based loss between each of the plurality of reconstructed signals from each of the plurality of decoder and an associated input signal from among the plurality of input signals; and
    validating the plurality of reconstructed signals pertaining to each of the plurality of input signals based on an associated plurality of parameters.

4. The method as claimed in claim 1, wherein the plurality of separable embeddings associated with each of the plurality of reconstructed signals are utilized to perform the plurality of downstream task with minimal finetuning of the encoder in the trained autoencoder network and, wherein the plurality of downstream task comprises automated sleep-stage

classification.

5. A system (100) comprising:
at least one memory (104) storing programmed instructions; one or more Input /Output (I/O) interfaces (112); and one or more hardware processors (102) operatively coupled to the at least one memory (104), wherein the one or more hardware processors (102) are configured by the programmed instructions to:

receive a signal from a sensor based wearable device worn by a subject under test for monitoring health conditions, wherein the received signal is a mixed signal comprising a plurality of neuro-physiological signals;
generate an encoded joint embedding of the received signal using a single encoder associated with a trained autoencoder network, wherein the single encoder comprises a plurality of convolution blocks, wherein each of the plurality of convolution blocks comprises a plurality of convolutions followed by batch normalizations, activation layers, and a pooling layer;
generate a final separable embedding based on an aggregated temporal context associated with the received signal by passing the encoded joint embeddings through additional convolutions, a Multi-Head Attention (MHA) mechanism and a feed forward layer associated with the trained autoencoder network, wherein the feed forward layer uses residual connections and layer normalizations; and
generate a plurality of reconstructed signals comprising at least one of a plurality of neural signals and a plurality of physiological signals based on the final separable embedding of the received signal using a multiple decoder framework associated with the trained autoencoder network, by:

generating a plurality of decoded signals by performing linear up sampling followed by a plurality of convolutions, batch normalizations and activations based on the final separable embedding using a plurality of decoders associated with the multiple decoder framework;
generating a plurality of potential reconstructed signals based on the plurality of decoded signals using additional convolution layers associated with the multiple decoder framework;
predicting a residual error associated with each of the plurality of the potential reconstructed signals using final convolution layers associated with the multiple decoder framework; and
generating the plurality of reconstructed signals by adding an associated predicted residual error to each of the plurality of potential reconstructed signals.

6. The system as claimed in claim 5, wherein the final separable embedding comprises a plurality of separable embeddings pertaining to each of the plurality of neuro-physiological signals.

7. The system as claimed in claim 5, wherein the trained autoencoder network comprises the single encoder with the MHA and the feed forward layer and a multiple decoder framework comprising the plurality of decoders and convolution layers, wherein steps for training the autoencoder network comprises:

initializing training of the autoencoder network with a plurality of skip connections between the single encoder and the plurality of decoders until a predefined number of training epochs to learn an encode function to encode a plurality of input signals into an associated plurality of separable embeddings, wherein the plurality of input signals are mixed signals comprising the plurality of neuro-physiological signals;
training the autoencoder network until a predefined number of training epochs to learn a plurality of decode functions to map the associated plurality of separable embeddings to a plurality of input signals, wherein the plurality of skip connections between single encoder and the plurality of decoders decays for a predefined number of epochs as the training progresses, wherein decaying of skip connections is performed by increasing dropout leading to minimal skip connections within the first few epochs and, wherein the decaying of skip connections allows the autoencoder to learn high frequency information for the reconstruction of the plurality of input signals and also producing accurate representations of the input signals in the respective encoded embeddings;
training autoencoder network to generate a plurality of reconstructed signals associated with the plurality of input signals based on the associated plurality of separable embeddings using a multimodal reconstruction loss, wherein the multimodal reconstruction loss is a weighted sum of smooth L1-loss and a correlation-based loss between each of the plurality of reconstructed signals from each of the plurality of decoder and an associated input signal from among the plurality of input signals; and
validating the plurality of reconstructed signals pertaining to each of the plurality of input signals based on an associated plurality of parameters.

8. The system as claimed in claim 5, wherein the plurality of separable embeddings associated with each of the plurality

of reconstructed signals are utilized to perform the plurality of downstream task with minimal finetuning of the encoder in the trained autoencoder network and, wherein the plurality of downstream task comprises automated sleep-stage classification.

**9.** One or more non-transitory machine-readable information storage mediums comprising one or more instructions which when executed by one or more hardware processors cause:

receiving a signal from a sensor based wearable device worn by a subject under test for monitoring health conditions, wherein the received signal is a mixed signal comprising a plurality of neuro-physiological signals;
generating an encoded joint embedding of the received signal using a single encoder associated with a trained autoencoder network, wherein the single encoder comprises a plurality of convolution blocks, wherein each of the plurality of convolution blocks comprises a plurality of convolutions followed by batch normalizations, activation layers, and a pooling layer;
generating a final separable embedding based on an aggregated temporal context associated with the received signal by passing the encoded joint embeddings through additional convolutions, a Multi-Head Attention (MHA) mechanism and a feed forward layer associated with the trained autoencoder network, wherein the feed forward layer uses residual connections and layer normalizations; and
generating a plurality of reconstructed signals comprising at least one of a plurality of neural signals and a plurality of physiological signals based on the final separable embedding of the received signal using a multiple decoder framework associated with the trained autoencoder network, by:

generating a plurality of decoded signals by performing linear up sampling followed by a plurality of convolutions, batch normalizations and activations based on the final separable embedding using a plurality of decoders associated with the multiple decoder framework;
generating a plurality of potential reconstructed signals based on the plurality of decoded signals using additional convolution layers associated with the multiple decoder framework;
predicting a residual error associated with each of the plurality of the potential reconstructed signals using final convolution layers associated with the multiple decoder framework; and
generating the plurality of reconstructed signals by adding an associated predicted residual error to each of the plurality of potential reconstructed signals.

**10.** The one or more non-transitory machine readable information storage mediums as claimed in claim 9, wherein the final separable embedding comprises a plurality of separable embeddings pertaining to each of the plurality of neuro-physiological signals.

**11.** The one or more non-transitory machine readable information storage mediums as claimed in claim 9, wherein the trained autoencoder network comprises the single encoder with the MHA and the feed forward layer and a multiple decoder framework comprising the plurality of decoders and convolution layers, wherein steps for training the autoencoder network comprises:

initializing training of the autoencoder network with a plurality of skip connections between the single encoder and the plurality of decoders until a predefined number of training epochs to learn an encode function to encode a plurality of input signals into an associated plurality of separable embeddings, wherein the plurality of input signals are mixed signals comprising the plurality of neuro-physiological signals;
training the autoencoder network until a predefined number of training epochs to learn a plurality of decode functions to map the associated plurality of separable embeddings to a plurality of input signals, wherein the plurality of skip connections between single encoder and the plurality of decoders decays for a predefined number of epochs as the training progresses, wherein decaying of skip connections is performed by increasing dropout leading to minimal skip connections within the first few epochs and, wherein the decaying of skip connections allows the autoencoder to learn high frequency information for the reconstruction of the plurality of input signals and also producing accurate representations of the input signals in the respective encoded embeddings;
training autoencoder network to generate a plurality of reconstructed signals associated with the plurality of input signals based on the associated plurality of separable embeddings using a multimodal reconstruction loss, wherein the multimodal reconstruction loss is a weighted sum of smooth L1-loss and a correlation-based loss between each of the plurality of reconstructed signals from each of the plurality of decoder and an associated input signal from among the plurality of input signals; and
validating the plurality of reconstructed signals pertaining to each of the plurality of input signals based on an associated plurality of parameters.

**12.** The one or more non-transitory machine readable information storage mediums as claimed in claim 9, wherein the plurality of separable embeddings associated with each of the plurality of reconstructed signals are utilized to perform the plurality of downstream task with minimal finetuning of the encoder in the trained autoencoder network and, wherein the plurality of downstream task comprises automated sleep-stage classification.

100
108
MEMORY 104
MODULES 106
REPOSITORY 110
I/O INTERFACE
112
HARDWARE
PROCESSORS
102

FIG. 1A

Decaying Skip
Mixed @100Hz
3000×1 (T×Ch)
Multi-Head
Self-Attention
Feed
Forward
A
Encoder Blocks
×2
Conv1D
BN
GELU
AvgPool
×3
Conv1D
BN
GELU
Decoder Blocks
×2
Ch+Ch'
Ch'
Ch
T
T'
Stack
Upsample
Conv1D
BN
Leaky
ReLU
Conv
1D
TanH

FIG. 1B

A
Separable
Embeddings
BN
EEG @100Hz
3000×1
×3
Linear
BN
ReLU
Linear
Softmax
Sleep Stage
5 class
BN
ECG @100Hz
3000×1


FIG. 1C

200

receive a signal from a sensor based wearable device worn by a subject under test for monitoring health conditions, wherein the received signal is a mixed signal comprising a plurality of neuro-physiological signals (202)

generate an encoded joint embedding of the received signal using a single encoder associated with a trained autoencoder network, wherein the single encoder comprises a plurality of convolution blocks, wherein each of the plurality of convolution blocks comprises a plurality of convolutions followed by batch normalizations, activation layers, and a pooling layer (204)

generate a final separable embedding based on an aggregated temporal context associated with the received signal by passing the encoded joint embeddings through additional convolutions, a Multi-Head Attention (MHA) mechanism and a feed forward layer associated with the trained autoencoder network, wherein the feed forward layer uses residual connections and layer normalizations (206)

generate a plurality of reconstructed signals comprising at least one of a plurality of neural signals and a plurality of physiological signals based on the final separable embedding of the received signal using a multiple decoder framework associated with the trained autoencoder network, by: (i) generating a plurality of decoded signals by performing linear up sampling followed by a plurality of convolutions, batch normalizations and activations based on the final separable embedding using a plurality of decoders associated with the multiple decoder framework (ii) generating a plurality of potential reconstructed signals based on the plurality of decoded signals using additional convolution layers associated with the multiple decoder framework (iii) predicting a residual error associated with each of the plurality of the potential reconstructed signals using final convolution layers associated with the multiple decoder framework; and (iv) generating the plurality of reconstructed signals by adding an associated predicted residual error to each of the plurality of potential reconstructed signals. (208)

**FIG. 2**

$10^{-1}$
$10^{-2}$
$\mathcal{L}_{rec}$
train
eval
0
5
10
15
20
25
30
35
40
Epochs


FIG. 3A

0.725
0.700
0.675
0.650
0.625
0.600
0.575
0.550
Accuracy
0
10
20
30
40
50
60
70
80
90
Epochs
train
eval

FIG. 3B

0.4
0.2
0.0
−0.2
−0.4
−0.6
−0.8
ECG
True
Predicted
0
500
1000
1500
2000
2500
3000
Samples

FIG. 3C

True
Predicted
EEG
0.4
0.2
0.0
−0.2
−0.4
0
500
1000
1500
2000
2500
3000
Samples

FIG. 3D

True
Predicted
ECG PSD
0.0020
0.0015
0.0010
0.0005
0.0000
0
5
10
15
20
25
30
Frequency (Hz)

FIG. 3E

0.014
0.012
0.010
0.008
0.006
0.004
0.002
0.000
EEG PSD
0
5
10
15
20
25
30
Frequency (Hz)
True
Predicted

FIG. 3F

Europäisches
Patentamt
European
Patent Office
Office européen
des brevets

# EUROPEAN SEARCH REPORT

Application Number
EP 25 22 5559

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X,P | DATTA SHREYASI ET AL: "Reconstruction of EEG and ECG from Single Channel Mixture using Branched Autoencoder based Separable Representations", ICASSP 2025 - 2025 IEEE INTERNATIONAL CONFERENCE ON ACOUSTICS, SPEECH AND SIGNAL PROCESSING (ICASSP), 7 March 2025 (2025-03-07), pages 1-5, XP093395103, DOI: 10.1109/icassp49660.2025.10887867 * the whole document * ----- | 1-12 | INV. A61B5/369 A61B5/00 |
| A,D | CHUANG CHUN-HSIANG ET AL: "IC-U-Net: A U-Net-based Denoising Autoencoder Using Mixtures of Independent Components for Automatic EEG Artifact Removal", NEUROIMAGE ELSEVIER, AMSTERDAM, NL, vol. 263, 27 August 2022 (2022-08-27), XP087221904, ISSN: 1053-8119, DOI: 10.1016/J.NEUROIMAGE.2022.119586 [retrieved on 2022-08-27] * abstract * * page 3, left-hand column, paragraph 2 - paragraph 6 * * page 7, right-hand column, paragraph 3 - page 9, left-hand column, paragraph 1 * * figures 2,5 * ----- | 1-12 | TECHNICAL FIELDS SEARCHED (IPC) A61B |
| A | US 2022/044106 A1 (ISPIR MUSTAFA [US] ET AL) 10 February 2022 (2022-02-10) * paragraph [0003] - paragraph [0004] * * paragraph [0020] - paragraph [0021] * * paragraph [0029] - paragraph [0032] * * figures 1,2 * ----- -/-- | 1-12 | |

The present search report has been drawn up for all claims

1

| Place of search | Date of completion of the search | Examiner |
|---|---|---|
| Berlin | 6 May 2026 | Völlinger, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches
Patentamt
European
Patent Office
Office européen
des brevets

# EUROPEAN SEARCH REPORT

Application Number
EP 25 22 5559

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CHUN-HSIANG CHUANG ET AL: "ART: Artifact Removal Transformer for Reconstructing Noise-Free Multichannel Electroencephalographic Signals", ARXIV.ORG CORNELL UNIVERSITY LIBRARY, 201 OLIN LIBRARY CORNELL UNIVERSITY ITHACA, NY 14853, 11 September 2024 (2024-09-11), XP091880848, * abstract * * page 2, right-hand column, paragraph 3 * * page 3, right-hand column, paragraph 3 - page 6, left-hand column, paragraph 1 * * page 1 * ----- | 1-12 | |
| A | CHING FANG ET AL: "Promoting cross-modal representations to improve multimodal foundation models for physiological signals", ARXIV.ORG CORNELL UNIVERSITY LIBRARY, 201 OLIN LIBRARY CORNELL UNIVERSITY ITHACA, NY 14853, 21 October 2024 (2024-10-21), XP091915635, * abstract * * section 3.2 * * figure 1 * ----- | 1-12 | TECHNICAL FIELDS SEARCHED (IPC) |

The present search report has been drawn up for all claims

1

| Place of search | Date of completion of the search | Examiner |
|---|---|---|
| Berlin | 6 May 2026 | Völlinger, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.........................................................................................................
& : member of the same patent family, corresponding document

EPO FORM 1503 03.82 (P04C01)

# ANNEX TO THE EUROPEAN SEARCH REPORT ON EUROPEAN PATENT APPLICATION NO. EP 25 22 5559

This annex lists the patent family members relating to the patent documents cited in the above-mentioned European search report. The members are as contained in the European Patent Office EDP file on
The European Patent Office is in no way liable for these particulars which are merely given for the purpose of information.

06-05-2026

| Patent document cited in search report | Publication date | Patent family member(s) | Publication date |
|---|---|---|---|
| US 2022044106 A1 | 10-02-2022 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- IN 202521017346